# EUROPEAN PATENT APPLICATION

(11) **EP 1 639 993 A1**
(43) Date of publication of application: **29.03.2006**
(21) Application number: 04745967.2
(22) Date of filing: 16.06.2004
(51) Int. Cl.: A61K 8/40, A61K 8/41, C11D 3/20, C11D 3/37, C11D 3/50

(54) **SHAMPOO OR BODY DETERGENT COMPOSITION**

(30) Priority: 17.06.2003 JP 2003171752
(71) Applicant: Takasago International Corporation, Tokyo-to 144-8721 (JP)
(72) Inventor: SEKINE, Miyuki, c/o CENTRAL RESEARCH LABORATORY, Hiratsuka-shi, Kanagawa (JP); KUMAMOTO, Hiroyasu, c/o CENTRAL RESEARCH LAB., Hiratsuka-shi, Kanagawa (JP)
(74) Representative: Neidl-Stippler, Cornelia
(86) International application number: PCT/JP2004/008425
(87) International publication number: WO 2004/112735

(57) **Abstract**

A shampoo or body detergent composition which comprises (A) a refrigerant substance selected from menthol, menthone, camphor, pulegol, isopulegol, cineole, Japanese peppermint oil, peppermint oil, spearmint oil, and eucalyptus oil, (B) a substance giving a cool feeling and comprising an N-substituted p-menthane-3-carboxamide derivative, (C) an anionic surfactant, and (D) a water-soluble polymer and/or polyhydric alcohol. It optionally contains (E) a substance which gives a warm feeling. The composition is satisfactory in the effect of giving a refrigerant feeling and cool feeling during use and in the retention of that effect thereafter.

## Description

### Technical Field

The invention relates to a shampoo or body detergent composition, further in more detail, relates to a shampoo or body detergent composition having an excellent and long-lasting cool and refreshing feeling effect.

### Background Art

With the recent drastic changes in consumers' life style and needs, there arises a trend toward daily used various products such as cosmetics, hair care products, toiletry products, bathing agents, and medicaments having not only the inherent function requisite for the products but also an additional function of imparting a cool and refreshing feeling during and after use. Therefore, various products which impart a cool and refreshing feeling have been available in various commodity forms. In many cases, a cool and refreshing feeling can be obtained by adding a cool feeling effect. Typical products with a preference for imparting a cool and refreshing feeling or cooling effect include cosmetics to be used in summer, hair care goods such as shampoos, rinses and hair conditioners, body care goods such as hand soaps and body shampoos, poultices, bathing agents and repellent sprays.

To produce the products having a cool and refreshing feeling or cooling effect, various cool and refreshing feeling agents or cool feeling substances such as menthol, camphor, methyl salicylate, menthyllactate, cineole, menthone, spearmint, peppermint, isopulegol, 3-menthoxypropane-1,2-diol, p-menthane-3,8-diol, and N-substituted p-menthane-3-carboxamide derivative have conventionally been compounded into the product. While the cool and refreshing feeling agent substances such as menthol, camphor, methyl salicylate, cineole, menthone, spearmint, peppermint and isopulegol can impart a strong cool and refreshing feeling, they have some problems, for example, when the additional amount of the cool and refreshing feeling agent substance is increased, the evaporation thereof causes a strong stimulus for eyes and nose, and the resultant persistency of the cool and refreshing feeling is short. On the other hand, as novel cool feeling substance, various compounds with p-menthane skeleton and analogues thereof such as menthyl lactate, 3-menthoxypropane-1,2-diol, p-menthane-3,8-diol, and N-substituted p-menthane-3-carboxamide derivatives were developed, and some of them are effective for foods and oral care goods. However they can not impart sufficient cool and refreshing feelings to hair wash goods and body detergents.

In order to correspond to the increasing demand on the market for these cool and refreshing feeling agent substances and cool feeling substances, there have been intensively performed researches and developments to provide novel cool feeling substances. Also, with regard to the cool and refresh feeling agents and cool feeling agents conventionally known, the combination of two or more thereof or the combination of the cool feeling agents and the other substances have been made for the purpose of enhancing a cooling feeling effect or improving a lasting effect of the cool feeling effect. Examples of the combination of the cool feeling substances and the other substances by which the improved cooling effect and persistency of the cool feeling effect are imparted include, for example, a combination of 3- (1-menthoxy) propane-1, 2-diol and particular glycerin ether (see the patent literature 1 below) and a combination of 3- (1-menthoxy) propane-1,2-diol and a hydrophilic poly ether modified-silicone (see the patent literature 2 below) . In addition, it has been proposed to imparting an excellent cool and refreshing feeling to, for example, a hair detergent composition comprising an amphoteric surfactant and a water-soluble quaternary nitrogen-containing polymer by a combination of specific cool feeling substances and specific warming and/or pungent substances (see the patent literature 3 below). Although these compositions show a somewhat increased cool feeling effect the resulted cool feeling effect is not yet sufficient. In addition, the durability of the cool feeling effect is not yet sufficient similarly. Therefore cool feeling substances having more excellent cooling effect and lasting effect of cooling effect are required.

From such view point, there are proposed a cool feeling agent composition using a specific cool feeling substance together with vanillyl butyl ether as a warming and/or pungent substance (see the patent literature 4 below) and a cooling effect-improved composition containing cool feeling substances of alcohols or ethers such as L-menthol, L-isopulegol, 3-(1-menthoxy)propane-,2-diol and paramenthane-3,8-diol and a cationic surfactant (see the patent literature 5 below). The compositions described in the former literature shows the improved cooling effect but the literature discloses no combined use of a water-soluble high-molecular-weight polymer or polyhydric alcohol and the cool feeling substance. In addition, the cool feeling agents are alcohols and ethers, and a very sufficient improving effect of the cooling effect might not always be shown. The compositions in the latter literature are ones for rinses or hair conditioners and the cationic surfactant is the essential component thereof. Further there has been proposed a scalp care agent composition comprising a warming and/or pungent substance and cationic polymer (see the patent literature 6 below). This composition just offers a high massage-like effect to the head skin and a lasting warming feeling, but there is no description about the cooling effect to the head skin.

In such background, concerning the shampoo and body detergent comprising a cool and refreshing feeling agent typified with L-menthol, there exists a strong desire to give it a strong cool and refreshing feeling or a cooling effect continuously with holding the additional amount of the cool and refreshing feeling agent low to control the stimulus based on the agent. However the conventional techniques have not been able to sufficiently respond to this desire.

On the other hand, there is proposed a method of applying N-substituted-p-menthane-3-carboxamid derivatives as the transdermal absorption promoting agent for shampoos in the patent literatures 7 and 8 below. The patent literatures disclose the combined use of the cool and refreshing feeling agents such as menthol, but it does not disclose about the cooling effect for the head skin or body skin. In addition, it is known that N-substituted-p-menthane-3-carboxamid derivatives are substances which in itself have a cool feeling action and are used as a refrigerant (see the patent literatures 9 and 10 below), but the effect of the combined use of the other cool feeling substances is not concretely disclosed.
Patent literature 1: JP-A-63-208505
Patent literature 2: JP-A-63-264522
Patent literature 3: JP-A-6-107527
Patent literature 4: JP-A-2000-44924
Patent literature 5: JP-A-2002-114649
Patent literature 6: JP-A-2000-191461
Patent literature 7: JP-A-2001-72605
Patent literature 8: JP-A-2001-58961
Patent literature 9: JP-A-47-16648
Patent literature 10: JP-A-8-283147

Accordingly, the object of the invention is to provide a shampoo or body detergent composition capable of imparting the improved cool and refreshing feeling or cooling effect when hair or body is washed and further of lasting its cooling effect thereafter.

### Disclosure of the Invention

After intensive studies and investigations concerning the method of giving an cool and refreshing feeling efficiently to a shampoo and a body detergent, the inventors have found that the shampoo or body detergent composition obtained by using a cool and refreshing feeling substance such as menthol together with N-substituted-p-menthane-3-carboxamid derivative as cool feeling substance and further compounding therewith an anionic surfactant and a water-soluble high molecular polymer and/or a polyhydric alcohol has an improved cool feeling effect, that is, an improved cool and refreshing feeling at hair or body washing, and an effectively lasting cool feeling effect, that is, a cool and refreshing feeling effect effectively lasting thereafter. Additionally, the inventors have also found that when a warming and/or pungent substance such as vanillyl alkyl ether is compounded into the composition above, an especially improved cool feeling effect that leads to an especially improved cool and refreshing feeling at hair or body washing and a lasting effect thereof after hair or body washing can be obtained. The invention has been accomplished based on these innovative findings.

The invention relates to the following shampoo or body detergent compositions described in following items [1] to [7].
[1] A shampoo or body detergent composition comprising following components (A), (B), (C) and (D);
   (A) one or more cool and refreshing feeling substances selected from the group which consists of menthol, menthone, camphor, pulegol, isopulegol, cineol, Japanese peppermint oil, peppermint oil, spearmint oil, and eucalyptus oil,
   (B) one or more cool feeling substances selected from the N-substituted-p-menthane-3-carboxamid derivatives represented by the following general formula (I):

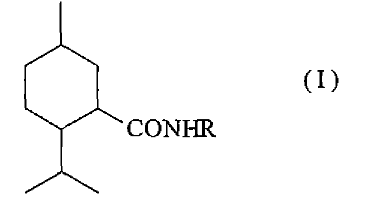

wherein R represents an alkyl group or alkenyl group having 1 to 10 carbon atoms,
(C) one or more components selected from anionic surfactants, and
(D) one or more components selected from water-soluble high-molecular-weight polymers and/or polyhydric alcohols.

[2] The shampoo or body detergent composition according to the above item [1], in which the component (A) is menthol.
[3] The shampoo or body detergent composition according to the above items [1] or [2] , in which the ratio of the cool and refreshing feeling substance of the component (A) to the cool feeling substance of the component (B) is (30:70) to (99:1) by weight.
[4] The shampoo or body detergent composition according to any one of the items [1] to [3] , in which the total amount of the cool and refreshing feeling substance of the component (A) and the cool feeling substance of the component (B) is from 0.001 weight-% to 2 weight-%.

[5] The shampoo or body detergent composition according to any one of the above items [1] to [4], in which one or more components selected from the warming and/or pungent substances of component (E) are used as additional components.

[6] The shampoo or body detergent composition according to any one of the above items [1] to [5], in which one or more cool feeling substances selected from p-menthane-3,8-diol, menthyl lactate, menthone glycerin ketal, monomenthyl succinate, alkali metal salt of monomenthyl succinate, menthoxyalkanol (wherein the number of the carbon atoms in the alkyl group is 2 to 6) , menthoxyalkyl ether (wherein the number of the carbon atoms in the alkyl group is 2 to 6), menthoxyalkandiol (wherein the number of the carbon atoms in the alkyl group is 3 to 6), and acyclic carboxamide derivatives are used as additional components.

[7] The shampoo or body detergent composition according to the above item [5] , in which the warming and /or pungent substance of the component (E) consists of one or more of vanillyl alkyl ether (wherein the number of the carbon atoms in the alkyl group is 1 to 6), 4-(1-menthoxymethyl)-2-(3',4'-dihydroxyphenyl)-1,3-dioxolane, 4-(1-menthoxymethyl)-2-(2'-hydroxy-3'-methoxyphenyl)-1,3-dioxolane, vanillamide alkanate (wherein the number of the carbon atoms in the alkyl group is 7 to 12), vanilline alkylene glycol acetal (wherein the number of the carbon atoms in the alkyl group is 3 to 6), ethylvanillin alkylene glycol acetal (wherein the number of the carbon atoms in the alkyl group is 3 to 6), capsaicin, dihydrocapsaicin, gingerol, cayenne pepper oil, cayenne pepper oleoresin, ginger oleoresin, nonylic acid vanillylamide, jambu oleoresin, zanthoxylum extract, sanshool I, sanshool II, zanthoxylum armatamide, black pepper extract, chavicine, piperine, and spilanthole.

### Detailed Explanation of the Invention

Hereinafter, the invention will be described more in detail.
A cool and refreshing feeling substance is used as the component (A) in the invention. Examples of the cool and refreshing feeling substance of the component (A) include menthol, menthone, camphor, pulegol, isopulegol, cineol, Japanese peppermint oil, peppermint oil, spearmint oil, eucalyptus oil, and the like. These all have a cooling effect and can give a cool and refreshing feeling, but the effects are different depending on the difference of geometric or optical structure thereof. These cool and refreshing feeling substances may be used alone or used in optional combination of two or more of them. It is especially preferable to use menthol as the main component of the cool and refreshing feeling substance, which is excellent in the cool and refreshing feeling-giving effect and easy to get, in the invention.

N-substituted-p-menthane-3-caboxamide derivatives represented by the above general formula (I) is used as the cool feeling substance of the component (B) in the invention. In the above general formula (I), R represents, for example, alkyl groups such as methyl group, ethyl group, n-propyl group, isopropyl group, n-butyl group, isobutyl group, and t-butyl group, and alkenyl groups such as ethynyl group, propenyl group, and buthynyl group; and methyl group, ethyl group, n-propyl group, and isopropyl group are especially preferable as R. These cool feeling substances may be used alone or in optional combination of two or more thereof.

N-substituted-p-menthane-3-carboxamide derivatives used as the component (B) in the invention is produced easily by the conventionally known method, for example, the method of reacting an appropriate amount of monosubstituted amine compound with an acid chloride obtained by the reaction of thionyl chloride with p-menthane 3-carbonic acid. The reaction between the acid chloride and the monosubstituted amine compound goes smoothly in the room temperature in the existence of, for example, alkali. The N-substituted-p-menthane-3-carboxamide derivatives above show geometric isomerism and optical isomerism. Depending on the raw materials used in the synthesis or the synthesis method, N-substituted-p-menthane-3-carboxamide derivative obtained is whether one kind of geometric isomer or optical isomer, or a mixture of geometric isomers or optical isomers. Every N-substituted-p-menthane-3-carboxamide derivatives shows a cooling effect, even though their effects are different each other by the difference of their geometric or optical structures.

The cool and refreshing feeling substance of the component (A) and the cool feeling substance of the component (B) of the invention may be used at any ratio to an extent of causing no adverse effects of the invention and the ratio of the component (A) and the component (B) used is preferably in the range of (1: 99) to (70:30) by weight. When the using ratio of the cool and refreshing feeling substance of the component (A) and the cool feeling substance of the component (B) is selected in this range, there is obtained a shampoo or body detergent composition which has low controlled stimulus of the cool and refreshing feeling substance, an excellent cool and refreshing feeling effect, and a long-lasting cool and refreshing feeling. The ratio of the component (A) and the component (B) is selected more preferably in the range of (70:30) to (95:5) by weight.

The amounts of the cool and refreshing feeling substance of component (A) and the cool feeling substance of component (B) in the shampoo or body detergent composition of the invention are properly decided according to the form and/or purpose of the shampoo or body detergent composition in use, that is the type of the final products thereof, for example, shampoo, rinse-in-shampoo, body shampoo, hand soap, facial soap, rinse, hair conditioner, hair treatment, and hair packs. The ratio of the total amount of the components (A) and (B) (hereinafter "the components (A) and (B)" would refer to "the components (A) + (B) mixed cool feeling substance".) to the whole amount of the composition is preferably selected in the rage of 0.001 weight-% to 2 weight-% considering the stability of the combined components (A) and (B) and the cooling effect.

The anionic surfactant used as the component (C) in the invention may be any anionic surfactant, and may be used alone or in optional combination of two or more thereof. Examples of the anionic surfactant of the invention include alkali metal salt, ammonium salt, amine salt, aminoalcohol salt and magnesium salt of alkyl sulfate, alkyl ether sulfate, alkylamide ether sulfate, alkyl aryl polyether sulfate, monoglyceride sulfate, alkyl sulfonic acid, alkylamide sulfonic acid, alkyl aryl sulfonic acid, olefin sulfonic acid, paraffin sulfonic acid, alkyl sulfosuccinic acid, alkyl ether sulfosuccinic acid, alkyl amide sulfosuccinic acid, alkyl succinamide acid, alkyl sulfoacetic acid, alkyl phosphate, alkyl ether phosphate, acyl sarcosine, acyl isethionate, and N-acyl taurine; salt of fatty acid; cocoanut oil acid or hydrogenated coconut oil acid, acyl lactylate, alkyl-D-galactosiduronic acid and the salts thereof; and polyoxyalkylene ether carbonic acid. And the anionic surfactant of the invention is not particularly limited to the above-exemplified anionic surfactants.

The ratio of the amount of the anionic surfactant of the component (C) to the whole amount of the shampoo or body detergent composition of the invention is properly adjusted depending on the type of the products, for example, shampoo, rinse-in-shampoo, body shampoo, hand soap, and facial soap. Ordinarily, it is from 5 weight-% to 40 weight-%, preferably from 5 weight-% to 30 weight-%, and especially preferably from 10 weight-% to 20 weight-%. The reason is that a sufficient washing ability can not obtained when the ratio is unless 5 weight-% and an appropriate viscosity can not obtained when the ratio is over 40 weight-%.

In the invention, there are used a water-soluble high-molecular-weight polymer [the component (D-1)] and /or a polyhydric alcohol [the component (D-2) as the component (D). As the water-soluble high-molecular polymer, any water-soluble high-molecular-weight polymers may be used in the invention as long as it is a water-soluble high-molecular-weight polymer, and the preferable water-soluble high-molecular-weight polymer is one having a cationic group. The water-soluble high-molecular-weight polymer of the component (D-1) is contained for giving the treatment effect, and the polyhydric alcohol of the component (D-2) is used for giving the moisturizing effect to the shampoo and body detergents of the invention.

As the aforementioned water-soluble high-molecular-weight polymer having a cationic group, there are exemplified, for example,
(1) a quaternized or nonquaternized vinylpyrrolidone/dialkylamino alkylacrylate or methacrylate copolymer,
(2) cellulose ether derivatives having a quaternary ammonium group,
(3) cationic cellulose derivatives comprising cellulose polymer or a grafted derivative thereof by a water-soluble quaternary ammonium monomer,
(4) a quaternized polysuccharide,
(5) a polymer having a piperazine part and a liner or branched bivalent alkylene or hydroxyalkylene group optionally interrupted by one or more atom (s) such as oxygen atom, sulfur atom, and nitrogen atom, or aromatic or heterocyclic ring(s), or a oxidation and/or quaternized polymer thereof,
(6) water-soluble polyaminoamides optionally closs linked and/or alkylated,
(7) alkylated polyaminoamide derivatives obtained by the alkylation of polyaminoamide derivatives by a bifunctional agent which are produced by the condensation between polyalkylenepolyamines and polycarbonates,
(8) polymers obtained by the reaction with polyalkylene polyamine having two primary amine groups, at least one secondary amine group, and dicarboxylic acid,
(9) cyclic polymers of a methyldiallylamine or dimethyldiallyl ammonium having a molecular weight of 20,000 to 3,000,000,
(10) a crosslinked polymer of methacryloyloxyethyl trimethylammonium chloride,
(11) quaternary polyammonium polymers,
(12) homopolymers or copolymers obtained from esters or amides of acrylic acid or methacrylic acid,
(13) a quaternary polymer of vinylpyrrolidone or vinylimidazole,
(14) polyamines,
(15) polyalkyleneimines,
(16) polymers having a vinylpyrrolidone part and a vinylpyridine part,
(17) condensates of polyamine and epichlorohydrin,
(18) polyureylenes, and
(19) polyamines, polyaminoamides, or quaternary polyammoniums selected from chitin derivatives.

The water-soluble high-molecular-weight polymer of the component (D-1) may be used alone or in combination of two or more thereof. The amount of the water-soluble high-molecular-weight polymer of the component (D-1) in the shampoo or body detergent composition of the invention is properly adjusted depending on the type of the products used, for example, shampoo, rinse-in-shampoo, body shampoo, hand soap, facial soap, rinse, hair conditioner, hair treatment, or hair packs. Ordinarily, the amount of the component (D-1) combined is preferably in the rage of 0.01 weight-% to 5 weight-%, more preferably 0.05 weight-% to 2 weight-%, and especially preferably 0.1 weight-% to 1 weight-% based on the amount of the whole composition. When the amount of the water-soluble high-molecular-weight polymer in the shampoo or body detergent composition is unless 0.01 weight-%, there might occur a problem that a treatment effect can not be given, when the amount of the water-soluble high-molecular-weight polymer in the shampoo or body detergent composition is over 5 weight-%, there might occur a problem that a undesirable stiffness of the hair and body is given after shampooing or body-washing.

As the polyhydric alcohol of the component (D-2) of the invention, any polyhydric alcohol may be used. Specific examples of the polyhydric alcohol include bivalent alcohols such as ethylene glycol, propylene glycol, isoprene glycol, 1,3-butylene glycol, 1, 2-butylene glycol, tetramethylene glycol, pentamethylene glycol, 2-butene-1,4-diol, hexylene glycol, and octylene glycol, trivalent alcohols such as glycerin, trimethylol propane, and 1,2,6-hexanetriol, tetravalent alcohol such as pentaerythritol, pentavalent alcohol such as xylitol, hexavalent alcohol such as sorbitol and mannitol, polyhydric alcohol polymers such as diethylene glycol, dipropylene glycol, triethylene glycol, polypropylene glycol, tetraethylene glycol, diglycerin, polyethylene glycol, triglycerin, tetraglycerin, and polyglycerin. Of these, glycerin, propylene glycol, and 1,3-butylene glycol are especially preferred.

The polyhydric alcohol of the component (D-2) may be used alone or in optional combination of two or more thereof. The amount of the polyhydric alcohol of the component (D-2) in the shampoo or body detergent composition of the invention may be properly adjusted depending on the type of the products in which the polyhydric alcohol of the component (D-2) is combined, for example, shampoo, rinse-in-shampoo, body shampoo, hand soap, facial soap, rinse, hair conditioner, hair treatment, and hair packs. Generally, the ratio of the total amount of the anionic surfactant of the component (C) and the used other surfactants to the amount of the polyhydric alcohol of the component (D-2), that is the ratio of surfactant/polyhydric alcohol is ordinarily selected in the range of 80/20 to 99/1, preferably in the rage of 85/15 to 98/2, more preferably in the range of 90/10 to 96/4. When the amount of the polyhydric alcohol is less than the aforementioned ratio, there might occur a problem that no moisturizing effect can be given. On the other hand when the amount of the polyhydric alcohol in the shampoo or body detergent composition is more than the aforementioned ratio, there might occur a problem that a sticky touch is given to the hair and body after use of the composition.

One or more cool feeling substances selected from p-menthane-3,8-diol, menthyl lactate, menthone glycerin ketal, monomenthyl succinate, alkali metal salt of the monomenthyl succinate, menthoxyalkanol (wherein the number of the carbon atoms in the alkyl group is 2 to 6), menthoxyalkyl ether (wherein the number of the carbon atoms in the alkyl group is 2 to 6), menthoxyalkandiol (wherein the number of the carbon atoms in the alkyl group is 3 to 6), and acyclic carboxamide derivatives, may be added into the shampoo or body detergent composition of the invention as additional components. An improved cool feeling effect can be given to the shampoo or body detergent composition by adding these additional components. In the alkali metal salts of the monomenthyl succinate, examples of the alkali metal therein include, for example, sodium and potassium. In the menthoxy alkanol and menthoxy alkyl ether, examples of the alkyl groups having 2 to 6 carbon atoms therein include, for example, ethyl group, n-propyl group, isopropyl group, n-butyl group, isobutyl group, t-butyl group, pentyl group, and hexyl group. In the menthoxyalkandiol, the alkyl groups having 3 to 6 carbon atoms include n-propyl group, isopropyl group, n-butyl group, isobutyl group, t-butyl group, pentyl group, and hexyl group. Specific examples of the acyclic carboxy imide derivatives include N-methyl-2,2-isopropylmethyl-3-methylbutane amide. These cool feeling substances may be used alone or in optional combination of two or more thereof.

In the invention, the warming and/or pungent substances of the component (E) may be added into the shampoo or body detergent composition of the invention as one of the additional components. The addition of the warming and/or pungent substance further improves the cool feeling effect and increases the cool and refreshing feeling of the composition of the invention. Further, the addition of the warming and/or pungent substance can be given not only the increased cool feeling effect, but also the increased blood circulation feeling in the scalp and skin and the increased drug activity feeling. As the warming and/or pungent substance of the component (E), any warming and/or pungent substance conventionally known may be used. Examples of the warming and/or pungent substance include, for example, vanillyl alkyl ether (wherein the number of the carbon atoms in the alkyl group is 1 to 6), 4-(1-menthoxymethyl)-2-(3',4'-dihydroxyphenyl)-1,3-dioxolane, 4-(1-menthoxymethyl)-2-(2'-hydroxy-3'-methoxyphenyl)-1,3-dioxolane, vanillamide alkanate (wherein the number of the carbon atoms in the alkyl group is 7 to 12) , vanilline alkylene glycol acetal (wherein the number of the carbon atoms in the alkyl group is 3 to 6), ethylvanillin alkylene glycol acetal (wherein the number of the carbon atoms in the alkyl group is 3 to 6), capsaicin, dihydrocapsaicin, gingerol, cayenne pepper oil, cayenne pepper oleoresin, ginger oleoresin, nonylic acid vanillylamide, jambu oleoresin, zanthoxylum extract, sanshool I, sanshool II, zanthoxylum armatamide, black pepper extract, chavicine, piperine, and spilanthole. In the vanillyl alkyl ether, examples of the alkyl group having 1 to 6 carbon atoms therein include, for example, methyl group, ethyl group, n-propyl group, isopropyl group, n-butyl group, isobutyl group, t-butyl group, pentyl group, and hexyl group. In the vanillamide alkanate, examples of the alkyl group having 7 to 12 carbon atoms therein include, for example, heptyl group, octyl group, nonyl group, decyl group, undecyl group, and dodecyl group. In the vanilline alkylene glycol acetal and the ethylvanillin alkylene glycol acetal, examples of the alkylene group having 3 to 6 carbon atoms therein include, for example, propylene group, butylene group, pentylene group, and hexylene group. Of these, the vanillyl alkyl ether is preferred and vanillyl butyl ether is especially preferred. The warming and/or pungent substance may be used alone or in optional combination of two or more thereof. In the composition of the invention, any amount of the warming and/or pungent substance can be used to an extent of causing no warming and/or pungent effects by the addition of the warming and/or pungent substance. Generally, the amount of the warming and/or pungent substance is selected in the range of 0.001 to 2.0 times by weight, preferably in the range of 0.01 to 1.0 times by weight against the total amount of the cool and refreshing feeling substance of the component (A) and the cool feeling substance of the component (B).

If necessary, silicones may be added into the shampoo or body detergent composition of the invention as the component (F). The addition of the silicone can give a slick feeling to the hair. The preferred silicone is methyl polysiloxane represented by the general formula (II) below:

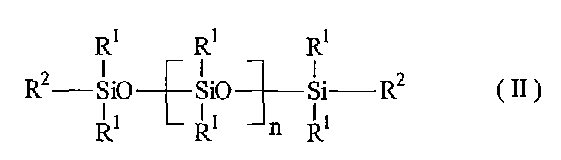

wherein R¹ represents methyl group or phenyl group, R² represents methyl group or hydroxyl group, and n is a number of 100 to 2000.

The silicones of the component (F) may be used alone or in combination thereof. The amount of the silicone added is generally 0. 01 weight-% to 10 weight-% to the amount of the whole composition, preferably 0.1 weight-% to 5 weight-%, and more preferably 0.5 weight-% to 3 weight-%.

Other than the aforementioned components, for example, a surfactant other than the aforementioned surfactants, an oil agent, a monohydric alcohol, a dye, a reducing agent, an oxidizing agent, a metal chelating agent, an antioxidant, a viscosity regulator, a preservative, an animal or plant extract, an anti-inflammatory, a fungicide, a dandruff inhibitor, an oxidation inhibitor, a pearlizing agent, an ultraviolet ray absorbing agent, a moisturizing agent, an organic or inorganic salt, a pH regulator, pigment, solvent, fragrance and capsules may be optionally combined in the composition of the invention.

The pH of the shampoo or body detergent composition of the invention is preferably selected in the range of 2 to 12, and especially in the range of 4 to 8.
The shampoo or body detergent composition of the invention is produced by the ordinary method and applied to, for example, shampoo detergent compositions such as shampoo, dry shampoo, rinse-in-shampoo, and conditioning shampoo, shampoo cosmetic compositions such as rinse, hair conditioner, hair treatment, and hair pack, body detergent compositions such as body shampoo, hand soap, and facial soap.

### Effect of Invention

When the shampoo or body detergent composition of the invention is used for a hair or body washing, it can give a stronger cool and refreshing feeling than ever with a little stimulus to the eyes and nose and can retain this excellent cool and refreshing feeling effect for a long time. That is, the shampoo or body detergent composition of the invention can give, with no stimulus to the eyes and nose, a comfortable chilly, refreshing and stimulating feeling to the head skin or the body skin while washing hair or body, and the very refreshing feeling lasts for a long time after the hair or the body is dried with a towel or the like.

### Best Mode for Carrying Out the Invention

Hereinafter, the invention will be described in more detail with reference to Examples which, however, do not limit the invention in any way.

### Example 1 and Comparative Example 1

Transparent shampoos of Example 1 and Comparative Example 1 were produced according to the following prescription shown in Table 1 by an ordinal method.

**Table 1 (Prescription of transparent shampoo)**

| | Example 1 | Comparative Example 1 |
|---|---|---|
| Polyquarternium-10¹⁾ | 10 | 10 |
| Sodium laureth sulfate ²⁾ (30 % aqueous solution) | 400 | 400 |
| Sodium cocoampho-acetate ³⁾ (40 % aqueous solution) | 100 | 100 |
| Cocamide DEA ⁴⁾ | 20 | 20 |
| 1,3-Butylene glycol | 20 | 20 |
| Citric acid | 3.5 | 3.5 |
| Sodium chloride | 1 | 1 |
| Methylparaben | 2 | 2 |
| Propylparaben | 1 | 1 |
| Tetrasodium edetate | 1 | 1 |
| Purified water | 431.5 | 431.5 |
| L-menthol | 4 | 5 |
| N-ethyl-p-menthane-3-carboxamide | 1 | - |
| Floral fragrance | 5 | 5 |
| Total | 1000 | 1000 |

| | | |
|---|---|---|
| 1) O-[2-hydroxy-3-(trimethylammonio)propyl]hydroxyethyl cellulose chloride | | |
| 2) Sodium polyoxyethylene lauryl ether sulfate | | |
| 3) Sodium coco-N-carboxyethyl-N-hydroxyethyl imidazolinium betaine | | |
| 4) Cocofatty acid diethanolamide | | |

### Test Example 1

A comparison concerning the cool and refreshing feelings just after shampooing and after hair-drying with towel was carried out using each 10 g of the shampoos of Example 1 and Comparative Example 1 by six panelists containing ordinary four men and two women through washing and drying their hair. The hair washings using the shampoos of Example 1 and Comparative Example 1 were done on the other day. The results are shown in Table 2.

**Table 2**

| | Shampoo of Example 1 has a stronger cool and refreshing feeling | Shampoo of Comparative Example 1 has a stronger cool and refreshing feeling | Both of the shampoos have the same cool and refreshing feeling |
|---|---|---|---|
| Just after shampooing | 5/6 | 0 | 1/6 |
| After toweling | 5/6 | 0 | 1/6 |

As shown in Table 2, five panelists among six answered that the shampoo of Example 1 has a stronger cool and refreshing feeling at both just after shampooing and after toweling than those of Comparative Example 1. It is apparent from this result that the shampoo or body detergent composition of the invention is more excellent in the strength and retainability of the cool and refreshing feeling than the comparative shampoo or body detergent composition.

### Example 2, Comparative Examples 2 and 3

Pearly shampoos of example 2, comparative examples 2 and 3 were produced according to the following prescription shown in Table 3 by an ordinal method.

**Table 3 (Prescription of pearly shampoo)**

| | Example 2 | Comparative Example 2 | Comparative Example 3 |
|---|---|---|---|
| Polyquarternium-10 | 5 | 5 | 5 |
| Sodium laureth sulfate (30 % aqueous solution) | 300 | 300 | 300 |
| Sodium laureth-3acetate (30 % aqueous solution) | 100 | 100 | 100 |
| Cocamide DEA | 40 | 40 | 40 |
| Glycol distearate | 10 | 10 | 10 |
| Citric acid | 2 | 2 | 2 |
| Sodium chloride | 5 | 5 | 5 |
| Methyl paraben | 2 | 2 | 2 |
| Propyl paraben | 1 | 1 | 1 |
| BHT ⁵⁾ | 0.5 | 0.5 | 0.5 |
| Tetrasodium edetate | 1 | 1 | 1 |
| Purified water | 527.5 | 527.5 | 527.5 |
| L-menthol | 2.5 | 3 | - |
| N-ethyl-p-menthane-3-carboxamide | 0.5 | - | 3 |
| Citrus fragrance | 3 | 3 | 3 |
| Total | 1000 | 1000 | 1000 |

| | | | |
|---|---|---|---|
| 5) 2,6-di-tert-butyl 4-methylphenol | | | |

### Test Example 2

A comparison concerning the cool and refreshing feelings just after shampooing was carried out using each 10g of the shampoos of Example 2 and Comparative Example 2 by eight panelists containing ordinary six men and two women through washing their hair. The hair washings using the shampoos of Example 2 and Comparative Example 2 were done on the other day. The results are shown in Table 4.

**Table 4**

| | Shampoo of Example 2 has a stronger cool and refreshing feeling | Shampoo of Comparative Example 2 has a stronger cool and refreshing feeling | Both shampoos have the same cool and refreshing feeling |
|---|---|---|---|
| Just after shampooing | 5/8 | 1/8 | 2/8 |

As shown in Table 4, five panelists among eight answered that the shampoo of Example 2 has a stronger cool and refreshing feeling at just after shampooing than that of Comparative Example 2. It is apparent from this result that the combined use of 1-menthol and N-ethyl-p-menthane-3-carboxamide can improve the cool and refreshing feeling while decrease the amount of stimulus 1-menthol used.

### Test Example 3

A comparison concerning the cool and refreshing feelings just after shampooing was carried out using each 10g of the shampoos of Example 2 and Comparative Example 3 by eight panelists containing ordinary six men and two women through washing their hair. The hair washings using the shampoos of Example 2 and Comparative Example 3 were done on the other day. The results are shown in Table 5.

**Table 5**

| | Shampoo of Example 2 has a stronger cool and refreshing feeling | Shampoo of Comparative Example 3 has a stronger cool and refreshing feeling | Both of the shampoos have the same cool and refreshing feeling |
|---|---|---|---|
| Just after shampooing | 8/8 | 0 | 0 |

As shown in Table 5, all of the eight panelists answered that the shampoo of Example 2 has a stronger cool and refreshing feeling. It is apparent from this result that the combined use of 1-menthol and N-ethyl-p-menthane-3-carboxamide can remarkably improve the strength of the cool and refreshing feeling compared with the case where only N-ethyl-p-menthane-3-carboxamide is used as the cool feeling substance.

### Examples 3, 4 and Comparative Example 4

Transparent shampoos of examples 3 and 4 and comparative example 4 were produced according to the following prescription shown in the table 6 by an ordinal method.

**Table 6 (Prescription of transparent shampoo)**

| | Example 3 | Example 4 | Comparative Example 4 |
|---|---|---|---|
| Polyquarternium-10 | 10 | 10 | 10 |
| Sodium laureth sulfate (30 % aqueous solution) | 300 | 300 | 300 |
| Sodium lauroylsarcosine (30 % aqueous solution) | 50 | 50 | 50 |
| Cocamide propylbetaine | 100 | 100 | 100 |
| Cocamide DEA | 40 | 40 | 40 |
| 1,3-Butyleneglycol | 20 | 20 | 20 |
| Citric acid | 3 | 3 | 3 |
| Methyl paraben | 2 | 2 | 2 |
| Propyl paraben | 0.5 | 0.5 | 0.5 |
| Disodium edetate | 1 | 1 | 1 |
| Purified water | 463.5 | 463.5 | 463.5 |
| L-menthol | 6.4 | 6.3 | 7 |
| N-ethyl-p-menthane-3-carboxamide | 0.6 | 0.6 | - |
| Vanillyl butyl ether | - | 0.1 | - |
| Citrus fragrance | 3 | 3 | 3 |
| Total | 1000 | 1000 | 1000 |

### Test Example 4

A comparison concerning the cool and refreshing feelings just after shampooing was carried out using each 10g of the shampoos of Example 3 and Comparative Example 4 by ten panelists containing ordinary ten men through washing their hair. The hair washings using the shampoos of Example 3 and Comparative Example 4 were done on the other day. The results are shown in the Table 7.

**Table 7**

| | Shampoo of Example 3 has a stronger cool and refreshing feeling | Shampoo of Comparative Example 4 has a stronger cool and refreshing feeling | Both of the shampoos have the same cool and refreshing feeling |
|---|---|---|---|
| Just after shampooing | 7/10 | 1/10 | 2/10 |

As shown in Table 7, seven panelists among ten answered that the shampoo of Example 3 has a stronger cool and refreshing feeling than that of comparative Example 4. It is apparent from this result that the combined use of 1-menthol and N-ethyl-p-menthane-3-carboxamide can improve the strength of the cool and refreshing feeling while decreases the amount of stimulus 1-menthol used.

### Test Example 5

A comparison concerning the cool and refreshing feelings just after shampooing was carried out using each 10g of the shampoos of Example 4 and Comparative Example 4 by ten panelists containing ordinary ten men through washing their hair. The hair washings using the shampoos of Example 4 and Comparative Example 4 were done on the other day. The results are shown in the Table 8.

**Table 8**

| | Shampoo of Example 4 has a stronger cool and refreshing feeling | Shampoo of Comparative Example 4 has a stronger cool and refreshing feeling | Both of the shampoos have the same cool and refreshing feeling |
|---|---|---|---|
| Just after shampooing | 8/10 | 1/10 | 1/10 |

As shown in Table 8, eight panelists among ten answered that the shampoo of Example 4 has a stronger cool and refreshing feeling than the shampoo of Comparative Example 4. The comments (which contain plural answers per one panelist) on the cool and refreshing feeling of the eight panelists who answered that the shampoo of Example 4 has a stronger cool and refreshing feeling are below.

The feeling is, if anything, a cool feeling; 2 panelists
The feeling is, if anything, a warming and/or pungent feeling; 6 panelists
The promoted blood circulation was felt; 5 panelists
A refreshing feeling was felt in the skin; 6 panelists
A drug activity feeling was felt; 4 panelists

It is apparent from this result that an additional use of vanillyl butyl ether together with 1-menthol and N-ethyl-p-menthane-3-carboxamide can give not only the cool and refreshing feeling but also the impression that the shampoo has pleasant effects for the skin comparison with the shampoo in which vanillyl butyl ether is not added.

### Example 5 and Comparative Example 5

Soap type body soaps of Examples 5 and Comparative Example 5 were produced according to the following prescription shown in the Table 9 by an ordinal method.

**Table 9 (Prescription of soap type body soap)**

| | Example 5 | Comparative Example 5 |
|---|---|---|
| Lauric acid | 25 | 25 |
| Myristic acid | 75 | 75 |
| Palmitic acid | 25 | 25 |
| Oleic acid | 25 | 25 |
| Lauroyl diethanolamide | 50 | 50 |
| Glycerin | 200 | 200 |
| Potassium hydroxide | 36 | 36 |
| Methyl paraben | 2 | 2 |
| BHT | 0.5 | 0.5 |
| Tetrasodium edetate | 1 | 1 |
| Purified water | 548.5 | 548.5 |
| L-menthol | 4 | 5 |
| N-ethyl-p-menthane-3-carboxamide | 1 | - |
| Citrus fragrance | 7 | 7 |
| Total | 1000 | 1000 |

### Test Example 6

A comparison concerning the cool and refreshing feelings just after body-washing was carried out using each 10g of the body soaps of Example 5 and Comparative Example 5 spread on a cotton towel by fifteen panelists containing ordinary six Japanese and nine Singapore through washing themselves. The body washings using the body soaps of Example 5 and Comparative Example 5 were done on the other day. The results are shown in Table 10.

**Table 10**

| | Body soap of Example 5 has a stronger cool and refreshing feeling | Body soap of Comparative Example 5 has a stronger cool and refreshing feeling | Both of the body soaps have the same cool and refreshing feeling |
|---|---|---|---|
| Just after body-washing | 12/15 | 2/15 | 1/15 |

As shown in Table 10, twelve panelists among fifteen answered that the body soap of Example 5 has a stronger cool and refreshing feeling than the body soap of comparative Example 5. It is apparent from this result that the combined use of 1-menthol and N-ethyl-p-menthane-3-carboxamide can improve the cool and refreshing feeling while decrease the amount of stimulus 1-menthol used.

### Example 6 and Comparative Example 6

Hand soaps of Examples 6 and Comparative Example 6 were produced according to the following prescription shown in Table 11 by an ordinal method.

**Table 11 (Prescription of hand soap)**

| | Example 6 | Comparative Example 6 |
|---|---|---|
| N-lauroyl triethanolamine L-glutamate (30 % aqueous solution) | 200 | 200 |
| Sodium N-lauroylmethyltaurine (30 % aqueous solution) | 100 | 100 |
| Triethanolamine laurate | 100 | 100 |
| Triethanolamine myristate | 100 | 100 |
| Laurylimidazorynium betaine | 50 | 50 |
| Lauroyl diethanolamide | 50 | 50 |
| Propylene glycol | 70 | 70 |
| Methylparaben | 2 | 2 |
| BHT | 0.5 | 0.5 |
| Tetrasodium edetate | 1 | 1 |
| Purified water | 316.5 | 316.5 |
| L-menthol | 6 | 7 |
| N-ethyl-p-menthane-3-carboxamide | 1 | - |
| Citrus fragrance | 3 | 3 |
| Total | 1000 | 1000 |

### Test Example 7

A comparison concerning the cool and refreshing feelings just after hand-washed was carried out in the steps including taking each 2g of the hand soaps of Example 6 and Comparative Example 6, washing the hands after the hand soap was formed up sufficiently on the both hands with properly supplied running water, then rinsing the washed hands perfectly by running water. The hand washing tests using the hand soaps of Example 6 and Comparative Example 6 were done in a distance of three hours by six panelists containing ordinary four men and two women. The results are shown in Table 12.

**Table 12**

| | Hand soap of Example 6 has a stronger cool and refreshing feeling | Hand soap of Comparative Example 6 has a stronger cool and refreshing feeling | Both of the hand soaps have the same cool and refreshing feeling |
|---|---|---|---|
| Just after hand-washed | 4/6 | 1/6 | 1/6 |

As shown in Table 12, four panelists among six answered that the hand soap of Example 6 has a stronger cool and refreshing feeling than that of Comparative Example 6. It is apparent from this result that the combined use of 1-menthol and N-ethyl-p-menthane-3-carboxamide can improve the cool and refreshing feeling while decrease the amount of stimulus 1-menthol used.

## Claims

1. A shampoo or body detergent composition comprising following components (A), (B), (C) and (D);
(A) one or more cool and refreshing feeling substances selected from the group which consists of menthol, menthone, camphor, pulegol, isopulegol, cineol, Japanese peppermint oil, peppermint oil, spearmint oil, and eucalyptus oil,
(B) one or more cool feeling substances selected from the N- substituted-p-menthane-3-carboxamid derivatives represented by the following general formula (I): 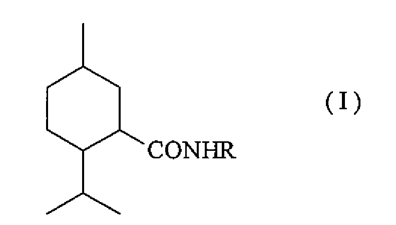 wherein R represents an alkyl group or alkenyl group having 1 to 10 carbon atoms,
(C) one or more components selected from anionic surfactants, and
(D) one or more components selected from water-soluble high-molecular-weight polymers and/or polyhydric alcohols.

2. The shampoo or body detergent composition according to claim 1, in which the component (A) is menthol.

3. The shampoo or body detergent composition according to claim 1 or 2, in which the ratio of the cool and refreshing feeling substance of the component (A) to the cool feeling substance of the component (B) is (30:70) to (99:1) by weight.

4. The shampoo or body detergent composition according to any one of claims 1 to 3, in which the total amount of the cool and refreshing feeling substance of the component (A) and the cool feeling substance of the component (B)is from 0.001 weight-% to 2 weight-%.

5. The shampoo or body detergent composition according to any one of claims 1 to 4, in which one or more components selected from the warming and/or pungent substances of component (E) are used as additional components.

6. The shampoo or body detergent composition according to any one of claims 1 to 5, in which one or more cool feeling substances selected from p-menthane-3,8-diol, menthyl lactate, menthone glycerin ketal, monomenthyl succinate, alkali metal salt of monomenthyl succinate, menthoxyalkanol (wherein the number of the carbon atoms in the alkyl group is 2 to 6), menthoxyalkyl ether (wherein the number of the carbon atoms in the alkyl group is 2 to 6), menthoxyalkandiol (wherein the number of the carbon atoms in the alkyl group is 3 to 6), and acyclic carboxamide derivatives are used in combination as additional components.

7. The shampoo or body detergent composition according to claim 5, in which warming and /or pungent substances of the component (E) consists of one or more of vanillyl alkyl ether (wherein the number of the carbon atoms in the alkyl group is 1 to 6), 4-(1-menthoxymethyl)-2-(3',4'-dihydroxypehnyl)-1,3-dioxolane, 4-(1-menthoxymethyl)-2-(2'-hydroxy-3'-methoxyphenyl)-I,3-dioxolane, vanillamide alkanate (wherein the number of the carbon atoms in the alkyl group is 7 to 12), vanilline alkylene glycol acetal (wherein the number of the carbons in the alkyl group is 3 to 6), ethylvanillin alkylene glycol acetal (wherein the number of the carbon atoms in the alkyl group is 3 to 6), capsaicin, dihydrocapsaicin, gingerol, cayenne pepper oil, cayenne pepper oleoresin, ginger oleoresin, nonylic acid vanillylamide, jambu oleoresin, zanthoxylum extract, sanshool I, sanshool II, zanthoxylum armatamide, black pepper extract, chavicine, piperine, and spilanthole.
